# FASCICULE DE BREVET EUROPEEN

(11) **EP 0 367 641 B1**
(45) Date de publication et mention de la délivrance du brevet: **21.09.1994**
(21) Numéro de dépôt: 89402632.7
(22) Date de dépôt: 26.09.1989
(51) Int. Cl.: C07K 13/00, A61K 37/02, A61K 37/24, C12N 15/19, A61K 39/395, C12N 5/12, C12N 15/85, C12N 5/10

(54) **Nouvelles lymphokines, séquences d'ADN codant pour ces lymphokines et compositions pharmaceutiques contenant ces lymphokines**
Lymphokine, DNS-Sequenzen, die diese Lymphokine kodieren, und sie enthaltende pharmazeutische Zusammensetzungen
Lymphokines, DNA sequences encoding these lymphokines and pharmaceutical compositions containing these lymphokines

(30) Priorité: 26.09.1988 FR 8812538
(43) Date de publication de la demande: 09.05.1990
(73) Titulaire: ROUSSEL-UCLAF, 75007 Paris (FR)
(72) Inventeur: Hercend, Thierry, F-94700 Maisons-Alfort (FR); Triebel, Frédéric, F-92200 Neuilly (FR)
(74) Mandataire: Le Guen, Gérard

(56) Documents cités:
- EP-A- 0 260 880
- EP-A- 0 320 806
- J.Exp. Med. 172, 1159-1163 (1990)
- fThe Journal of experimental Medecine, Vol. 165, mars 1987, pages 601-614, The Rockefeller University Press; J. Jongstra et al."The isolation and sequence of a novel gene from a human functional T cell line"
- The journal of Biochemistry, vol. 99, no. 3, 1986, pages 885-894, K. Obaru et al.,"A cDNA clone used to stdy mRNA inducible in human tonsillar lymphocytes by a tumor promoter"
- Proceedings of the national academy of sciences of the USA, vol 84, octobre 1987, pages 6995-6999, L.J. Emorine et al."Structure of the gene for human beta 2-adrenergic receptor: expression and promoter characterization"
- The Embo Journal vol. 7, n0 9, septembre 1988, pages 2807-2813, J.M. Salbaum et al.:The promoter of Alzheimers disease amyloid A4 precursor gene"

## Description

La présente invention concerne des lymphokines humaines, des séquences d'ADN codant pour ces lymphokines et les applications pharmaceutiques et biologiques de ces lymphokines.

Il est bien connu que le système immunitaire humain comprend des éléments cellulaires et des substances solubles secrétées par les cellules. Suivant leur origine ces substances solubles sont appelées lymphokines (si elles sont secrétées avant tout par les lymphocytes T) ou monokines (si elles sont secrétées avant tout par les monocytes). Ces "cytokines" (c'est-à-dire plus généralement les substances solubles secrétées par les cellules) ont un rôle de communication entre les éléments cellulaires du système immunitaire; elles peuvent par exemple en tant que facteur de croissance représenter un signal d'amplification de la réponse immunitaire : c'est le cas de l'interleukine-2, l'une des lymphokines la mieux étudiée. De plus, elles interviennent dans la communication entre le système immunitaire et les autres systèmes biologiques de l'organisme, par exemple le système hématopoïétique. Il est bien connu que les lymphokines ont une activité biologique, dite pléiotropique. De fait, toutes les lymphokines décrites jusqu'à présent ont des effets régulateurs sur de multiples types cellulaires. Enfin, les cytokines peuvent avoir aussi des fonctions effectrices directes; par exemple le "Tumor Necrosis Factor" est capable comme son nom l'indique d'induire la destruction de certaines cellules tumorales.

La découverte et la caractérisation des cytokines ont été rendues possibles par la mise au point des techniques de génie génétique. Cette méthodologie permet par diverses techniques expérimentales de caractériser les gènes codant pour des protéines et donc à travers la séquence nucléotidique du gène de connaître la séquence peptidique. D'autres applications de ces techniques de génie génétique fondées sur les mêmes principes expérimentaux permettent grâce à des systèmes d'expression procaryotes ou eucaryotes de produire en quantités virtuellement infinies les protéines dont le gène a été découvert.

Dans The Journal of Experimental Medecine, vol. 165, p. 601-614, Jongstra et al. et EP 320 806, est décrit le clonage d'un gène de lymphocytes T désigné 519. Selon cette publication ce gène coderait pour une protéine de 129 acides aminés. Ce produit ne serait toutefois pas une protéine secrétée et ne serait donc pas une lymphokine.

Les inventeurs ont cherché à découvrir de nouveaux gènes codant pour les lymphokines non décrites à ce jour.

Le développement des travaux des inventeurs a permis de cloner et de caractériser un nouveau gène, désigné LAG-2 (pour "Lymphocyte Activation Gene-2"), et une séquence d'ADN, désignée FC24, codant pour un polypeptide produit du gène. Ce polypeptide LAG-2 est secrété par les lymphocytes humains activés, après élimination d'une séquence signal, sous forme d'une protéine mature.

La présente invention a en conséquence pour objet une séquence d'ADN comprenant la séquence nucléotidique, désignée FC24, 
10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG
La traduction commence au nucléotide 25 et se termine au nucléotide 459 inclus.

La présente invention a également pour objet le polypeptide code par FC24 à savoir le polypeptide LAG-2 ayant la séquence suivante* 
5                10               15               20               25               30
M A T W A  L L L L A  A M L L G  N P G L V  F S R L S  P E Y Y D

35               40               45               50               55               60
L A R A H  L R D E E  K S C P C  L A Q E G  P Q G D L  L T K T Q

65               70               75               80               85               90
E L G R D  Y R T C L  T I V Q K  L K K M V  D K P T Q  R S V S N

95               100              105              110              115              120
A A T R V  C R T G R  S R W R D  V C R N F  M R R Y Q  S R V I Q

125              130              135              140              145              150
G L V A G  E T A Q Q  I C E D L  R L C I P  S T G P L
Les 22 premiers acides aminés devraient constituer une séquence signal qui est éliminée dans la protéine mature.
* La signification des symboles selon la nomenclature internationale est rappelée en annexe.

La présente invention a donc plus particulièrement pour objet le polypeptide ayant la séquence suivante : 
R L S P E Y Y D
L A R A H L R D E E K S C P C L A Q E G P Q G D L
L T K T Q E L G R D Y R T C L T I V Q K L K K M V
D K P T Q R S V S N A A T R V C R T G R S R W R D
V C R N F M R R Y Q S R V I Q G L V A G E T A Q Q
I C E D L R L C I P S T G P L
Il est connu que les gènes des cellules eucaryote présentent un phénomène de polytypie. Par ce phénomène, certains des amino acides de la protéine codée sont parfois remplacés sans modification de l'activité. La présente invention englobe les polypeptides provenant de ce phénomène.

La présente invention a donc plus généralement pour objet une lymphokine ayant la séquence peptidique : 
X - R L S  P E Y Y D  L A R A H  L R D E E  K S C P C
L A Q E G  P Q G D L  L T K T Q  E L G R D  Y R T C L
T I V Q K  L K K M V  D K P T Q  R S V S N  A A T R V
C R T G R  S R W R D  V C R N F  M R R Y Q  S R V I Q
G L V A G  E T A Q Q  I C E D L  R L C I P  S T G P L
dans laquelle X est choisi parmi H, un reste méthionine ou la séquence : M A T W A  L L L L A  A M L L G  N P G L V  F S
et les séquences qui en diffèrent par un ou plusieurs amino-acides et qui possèdent la même activité.

Les inventeurs ont en outre trouvé une séquence d'ADN qui est une région promotrice d'un gène codant pour une lymphokine selon l'invention. Cette séquence est la suivante : 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC
 
490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA

610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
La présente invention a en conséquence également pour objet cette séquence d'ADN.

La présente invention a également pour objet une séquence d'ADN comprenant une séquence d'ADN promotrice telle que définie ci-dessus et une séquence d'ADN codant pour une lymphokine selon la présente invention.

Dans la présente invention, les inventeurs ont tout d'abord obtenu un ADN complémentaire, FC24, par les opérations suivantes :
- culture de cellules lymphocytaires dites cellules cytotoxiques naturelles
- isolement à partir de ces lymphocytes de l'ARN messager lié aux membranes du réticulum endoplasmique intra-cellulaire
- obtention de l'ADN complémentaire simple brin à partir de l'ARN messager, puis de l'ADN complémentaire double brin
- insertion dans un vecteur tel que le bactériophage lambda GT10
- préparation d'une sonde d'ADN simple brin à partir de l'ARN messager des cellules et purification par une technique d'hybridation-soustraction de façon à sélectionner les copies des ARN présentes dans les cellules lymphocytaires et absentes dans d'autres cellules hématopoietiques
- sélection des ADN complémentaires insérés dans le vecteur qui réagissent avec la sonde
- transfert de l'ADNc sélectionné dans un vecteur plasmidique pour l'amplifier, le purifier et en réaliser le séquençage.

Les lymphokines selon l'invention peuvent être obtenues par synthèse peptidique classique ou par application de techniques de génie génétique comprenant l'insertion d'une séquence d'ADN codant pour une lymphokine selon l'invention dans un vecteur d'expression tel qu'un plasmide et la transformation de cellules avec ce vecteur d'expression.

La présente invention a donc également pour objet des plasmides et des vecteurs d'expressions comprenant une séquence d'ADN codant pour une lymphokine selon l'invention ainsi que des hôtes transformés avec ce vecteur.

La présente invention a également pour objet une composition pharmaceutique comprenant à titre de principe actif une lymphokine selon l'invention.

La présente invention a également pour objet des anticorps monoclonaux dirigés contre une lymphokine selon l'invention ou une séquence immunogène d'une telle lymphokine.

La présente invention a également pour objet des hybridomes produisant de tels anticorps monoclonaux.

La présente invention a également pour objet un procédé de dosage des lymphokines selon l'invention qui comprend l'utilisation d'anticorps monoclonaux selon l'invention.

A cet effet, on peut utiliser une méthode radioimmunologique de type RIA ou de type IRMA (technique de type sandwich utilisant un antigène froid et la compétition entre un anticorps froid et un anticorps marqué) ou une méthode immunoenzymatique de type ELISA ou de type IEMA (technique de type sandwich).

Les anticorps monoclonaux selon l'invention peuvent être préparés selon une technique classique. A cet effet les polypeptides peuvent être couplés si nécessaire à un agent immunogène, tel que l'anatoxine tétanique, par un agent de couplage tel que le glutaraldéhyde, un carbodiimide ou la benzidine bis diazotée.

On décrira ci-après plus en détail :
- l'obtention de l'ADNc FC24 codant pour le polypeptide LAG-2,
- l'obtention du gène LAG-2 et sa caractérisation,
- l'obtention de la protéine recombinante purifiée LAG-2 et l'obtention de la séquence peptidique NH₂-terminale.

### I - Culture et préparation de l'ARNm lié aux membranes du réticulum endoplasmique

L'isolement et les caractéristiques du clone foetal, F55IIIE5 (phénotype CD3⁻ CD2⁺) ont été décrite préalablement par Nowill et al (1).

La culture de masse a été effectuée en présence d'interleukine-2 recombinante, de surnageant de milieu conditionné lymphocytaire, sur une sous-couche nourricière de cellules sanguines mononuclées irradiées allogéniques et d'une lignée cellulaire transformée par le virus EBV (appelée LAZ 388) dans des plaques à fond en V à 96 trous. Les cellules ont été mises à 3000 cellules par puits au jour 0. Le recueil de 200 plaques à 3 x 10⁶ cellules par ml au jour 12 a permis de recueillir 6 x 10⁹ cellules.

La préparation des ARN cytoplasmiques, des ARN liés aux membranes du réticulum endoplasmique et des ARNm ont été faites en apportant certaines variantes aux méthodes décrites par Maniatis (2) Mechler (3) et Aviv (4). Ainsi 4 x 10⁹ cellules de F55IIIE5 ont été déposées sur des gradients de saccharose après choc hypotonique et broyage mécanique selon la méthode décrite par Mechler (3). Les ARN cytoplasmiques portés par les ribosomes liés aux membranes du réticulum endoplasmique ont été purifiés entre des gradients de saccharose. Ceci permet de travailler par la suite avec des ARNm qui ont une séquence signal et qui par conséquent codent pour des protéines portées par la membrane ou secrétées dans la partie interne de l'ergastoplasme, puis vers l'extérieur de la cellule. Cette méthode d'isolement de l'ARN de type dit MB (membrane-bound) permet d'emblée d'éliminer environ 90% des gènes transcrits qui codent pour des protéines intra-cellulaires ne pouvant pas être secrétées vers l'extérieur ou transportés vers la membrane et par conséquent sans intérêt dans le cadre de l'invention. En plus de l'isolement du ARNm MB-F55IIIE5 qui sert de substrat à d'une part la construction de la banque et d'autre part la fabrication de la sonde, les méthodes de purification décrites par Aviv (4), Maniatis (2) et Triebel (8) ont permis d'isoler des ARN de divers clones et lignées cellulaires qui sont utilisés au point V et des ARNm des cellules Jurkat, U937, Laz388 et K562 (environ 10⁹ cellules de chacune des lignées) qui sont utilisés pour soustraire la sonde.

Ces méthodes comprennent :

### A - Préparation de l'ARN cytoplasmique.

A une ampoule de 20 à 30.10⁶ cellules en culot sec, on ajoute 1 ml de tampon de lyse (Tris HCl 50mM, EDTA 62,5 mM, Triton X-100 0,4%, LiCl 2,5 M). Après dissolution douce du culot, on transfère le tampon de lyse dans les tubes Eppendorf froids, contenant 50 ul de NP40 10%.

Après 5 mn sur glace, on centrifuge 1 min à 8 K. On prélève le surnageant (ARN) et on l'ajoute dans les tubes Falcons contenant 1 ml de phénol, 1 ml de CHCl3, 1 ml de STE SDS 2% (NaCl 150 mM, Tris 10 mM, MgCl₂ 1 mM, 2% SDS). On centrifuge 10 mn à 5 K. On prélève la phase supérieure, on ajoute 1 ml de phénol et 1 ml de chloroforme. On centrifuge 5 mn à 5 K. On prélève la phase supérieure. On ajoute 100 µl EDTA 0,2 M, 200 µl NaAc 3M et 5 ml éthanol. On mélange et on laisse à - 20°C la nuit. On centrifuge 30 mn à 10 K. On sèche le culot. On reprend dans 400 µl de NaAc 0.3 M froid. On ajoute 1 ml d'éthanol dans le tube Falcon. On transfère l'éthanol dans l'Eppendorf, on mélange, on laisse 1h à - 20°C. On centrifuge 10 min à 13 K, aspire l'alcool et sèche. On ajoute 30 µl d'eau. On centrifuge, on congèle immédiatement à - 80 °C. On contrôle la dégradation et la quantité en mettant 1 µl sur gel dénaturant (1% agarose dans tampon TBE (Tris, Base, EDTA) pH 8,5 autoclavé (BET 1 µg/ml).

### B - Préparation de l'ARN messager lié aux membranes du réticulum endoplasmique

On reprend les cellules à 10⁸ cellules/ml dans du tampon hypotonique RSB (KCl 10mM, MgCl₂ 1,5 mM, Tris-HCl 10mM pH 7,4) à la température de la glace traité préalablement avec 0,1% DEPC. On laisse 5 min sur la glace. On rompt les cellules mécaniquement avec 10 coups d'homogéniseur de Dounce (type B). On centrifuge à 1000 g pendant 2 min pour sédimenter les noyaux. Le surnageant ou "extrait cytoplasmique" est ensuite utilisé pour la séparation ribosomes libres/extraits membranaires. 0,7 ml d'extrait cytoplasmique est mélangé avec 3,2 ml de tampon 2,5 M saccharose TK (Tris-HCl 0,05 M, pH 7,4, KCl 0,15 M, MgCl₂ 0,005 M) puis ce mélange est déposé sur 2 ml de 2,5 M saccharose TK. Puis on ajoute 8 ml de 2,05 M saccharose TK et ensuite 4 ml de saccharose 1,3 M TK. On fait tourner à 4°C pendant 5 h dans un rotor pendulaire type Spinco SW28 à 25.000 rmp. On pique avec une aiguille la bande compris entre le 2,05 M et le 1,3 M SAccharose. On ajoute un volume égal de TE 10 : 1 (Tris HCl 10mM, EDTA 1mM). On fait une extraction avec du phénol puis un mélange phénol-chloroforme. On précipite avec 1/10 de NaAc 3 M et 2,5 vol. d'éthanol.

Pour la sélection de l'ARN poly (A)⁺ on utilise une colonne d'oligo (dT)-cellulose comprenant 1,2 ml de gel équilibrée avec le tampon de charge : Tris-HCl 20mM (pH 7,6), Nacl 0,5 M, EDTA 1mM complété avec SDS. On lave avec une solution aqueuse de NaOH 0,1 M et EDTA 5mM. On lave avec 5 volumes de tampon de charge. On dissout l'ARN dans de l'eau et on chauffe à 65°C 5 min. On ajoute deux fois un volume identique de tampon de charge. On attend que la température s'équilibre. On récolte l'effluent. On chauffe à 65°C et on recommence. On lave la colonne avec 5 à 10 volumes de tampon de charge puis 4 volumes de tampon de charge NaCl 0,1 M. On élue le poly(A)⁺ avec 2-3 volumes de Tris-HCl (pH 7,5) 10mM, EDTA 1mM, SDS 0,05%.

On ajoute de l'acétate de sodium 3 M (pH 5,2) au 1/10. On précipite avec 2,2 vol. d'éthanol.

### II - Obtention de la banque d'expression

La préparation de l'ADN complémentaire simple brin à partir de l'ARN messager lié aux membranes du réticulum endoplasmique cellulaire de la cellule F55IIIE5 puis de l'ADN complémentaire double brin, l'addition de "linker" EcoRI, la ligation dans le site EcoRI du vecteur Lambda GT10, l'empaquetage in vitro ont été réalisés selon les techniques décrites par Gubler et al (5), et Huynh et al (6) en utilisant un kit de clonage commercial (Amersham Corp. Arlington Heights, II).

A cet effet on utilise 1 ug de ARNm lié aux membranes du reticulum endoplasmique. Environ 80 % de ce matériel a été transformé en ADNc simple brin.

### III - Préparation de la sonde d'ADN complémentaire

La préparation de la sonde d'ADN complémentaire simple brin est effectuée par soustraction par deux cycles d'hybridation sur un excès d'ARN messager des cellules dites "à éliminer" (Jurkat, Laz 388, U937, K562) suivi de passages sur colonnes d'hydroxyapatite, ce qui permet d'éliminer le complexe double brin ADNc-ARNm. Après 2 cycles d'hybridation et 2 passages sur colonne il reste environ 6-7% de la radioactivité, c'est-à-dire qu'environ 7% du matériel dit MB ("membrane-bound") F55IIIE5 n'existe pas dans les cellules tumorales Jurkat, K562,U937 et Laz 388. C'est ce matériel qui sert de sonde pour aller rechercher dans la banque MB-F55IIIE5 les ADNc correspondants. Cette technique utilise les conditions d'hybridation-soustraction décrites par Davis et al (7).

### Préparation de la sonde soustraite [MB-FSSIIES -ArNm de Jurkat, K562,Laz 388,U937]

A partir de 5 µg de ARNm MB-F55IIIE5 on prépare une sonde d'ADNc simple brin marquée au ³² P-dCTP (activité spécifique : 800 Ci/mmol⁻¹) dans un volume de 50 µl.

Après incubation de 2 h à 42°C avec l'enzyme transcriptase réverse, on ajoute 5 µl EDTA 0,2 M puis 50 µl NaOH 0,2 N. On incube à 65°C, 1 h. On ajoute 60 µl HCl 1N et 30 µl Tris-HCl (pH 8) 2 M. On ajoute 1/10^{e} vol. NaAc 3M. On ajoute 7 µl d'ARNm de chacune des 4 lignées tumorales pour précipiter la sonde, puis on ajoute 2,5 vol. d'éthanol.

On laisse 1 h à -20°C. On centrifuge, on lave à l'éthanol 70% et on sèche. On reprend dans 7,5 µl d'H₂O, on ajoute 7,5 µl NaH₂PO₄ 0,5, M pH 7, 1mM EDTA, 0,25% SDS. On incube à l'étuve 68°C pendant 20 heures.

On dilue le contenu dans 1 ml NaH₂PO₄ 0,12 M, 0,1% SDS. On charge sur une colonne d'hydroxyapatite équilibrée à 60°C dans le même tampon. L'effluent (matériel simple brin) est concentré par du sec-butanol et passé sur une colonne G-50 pour enlever le tampon phosphate. On ajoute de nouveau 7 µg de ARNm de chacune des lignées et on recommence l'hybridation et le passage sur colonne. Après ces 2 passages, on récupère 7% de la quantité de la radioactivité du départ.

### IV - Isolement et caractérisation des clones d'ADNc

L'hybridation avec la sonde et le criblage ont été effectuées comme décrit par Huynh (6) en utilisant des phases recombinants et la technique des filtres de Nylon. L'hybridation a été effectuée à 42°C avec préhybridation avec une solution d'hybridation de type Southern et addition de la sonde soustraite MB - F55IIIE5 simple brin à 5.10⁶ cpm/ml.

Environ 20.000 phages recombinants ont été criblés. Environ 120 pages positifs ont été obtenus avec la sonde soustraite décrite plus haut. Parmi ceux-ci 1 phage contenant un insert d'environ 200 paires de bases a été choisi puis amplifié dans pBS. Les inserts de ces phages ont été utilisés pour préparer une sonde marquée au ³²P comme décrit par Triebel et al. (8). 38 phages parmi les 120 phages positifs hybridaient avec cette sonde en réaction croisée. Les inserts de ces 38 phages ont permis de définir la famille LAG-2. Cette méthodologie est usuelle en biologie moléculaire (2).

La mise en culture des phages positifs, la purification des ADN correspondants, la purification des ADN complémentaires sous forme de fragments par excision à partir d'une électrophorèse sur gel d'agarose à point de gélification bas ont été réalisés selon les méthodes décrites par Maniatis(2) et Huynh(6).

### V - Amplification et clonage de l'ADNc le plus long

La ligation des ADN complémentaires les plus longs de la famille LAG-2 dans le vecteur plasmidique pBS digéré par l'endonucléase EcoRI et traité par la phosphatase alkaline d'intestin de veau, la transformation de bactéries JM 109 compétentes et le criblage des recombinants par un double système de sélection (ampicilline + X-gal/IPTG) ont été effectués selon des méthodes de génie génétique classiquement utilisées.

La purification et la préparation en quantité importante des ADN complémentaires recombinants clonés dans pBS ont été réalisés en utilisant la méthode de purification par gradient sur chlorure de césium décrite par Maniatis (2). complémentaire à tester ont été préparées à partir d'une préparation pure de plasmides recombinants.

L'ADNc le plus long est l'ADNc FC24 dont la séquence est l'un des objets de l'invention.

L'analyse de la taille et de la répartition cellulaire des différents transcripts (ARN messager) correspondant à chacune de ces familles, l'évaluation d'ensemble de la stratégie d'hybridation-soustraction par l'analyse de l'hybridation sur l'ARN messager des cellules Jurkat, Laz388, U937, K562, la détermination des familles intéressantes à distribution transcriptionnelle réduite ont pu être menées à bien grâce à la technique dite de "Northern blot" dont les conditions de réalisation ont été décrites par Triebel et al (8).

### VI - Séquençage de l'ADNc FC24

Le séquençage de l'ADN complémentaire FC24 a été réalisé grâce à la "ADN polymérase fragment large" (enzyme de Klenow) dans une réaction de terminaison des chaînes nucléotidiques par des didéoxynucléotides selon la méthode de Sanger, soit directement à partir du plasmide recombinant purifié (séquence de ADN double brin) soit indirectement après clonage dans le phage M13 (séquence d'ADN simple brin). Il faut noter que les ADNc ont été séquencés sur les 2 brins et que la séquence a été aussi établie après que les fragments de ces ADNc (EcoRI-Pst et Eco RI-Xba) ont été reclonés dans le plasmide pBS afin de faciliter l'interprétation des séquences faites sur de l'ADN double brin.

### VII - Analyses du type Northern blot de l'expression du gène LAG-2

L'extraction de l'ARN total intracytoplasmique a été réalisée selon Triebel et al. (8). L'ARN a été séparé sur gel d'agarose en présence de glyoxal et transféré sur une membrane de nylon toujours d'après Triebel et al. (8). L'hybridation de la membrane de nylon a été réalisée à 42° C en présence de 50 % de formamide avec la sonde FC24 obtenue par marquage de l'ADNc FC24 au ³²P et comme décrit par Triebel et al. (8).

Il n'y a pas d'expression du gène LAG-2 dans les lignées ayant servi à l'hybridation-soustraction comme les lignées Jurkat, Laz 388, K562 et U927. Il n'a pas été trouvé d'expression du gène LAG-2 dans des tissus extra-hémapoïétiques comme par exemple dans le cerveau ou dans une lignée d'hépatome. L'expression du gène LAG-2 est uniquement retrouvée sous la forme d'un message à environ 0,8 kb dans : 1) les lignées clonales ou polyclonales IL-2 dépendantes de type NK ou de type T ; 2) dans les lymphocytes du sang périphérique après stimulation par la phytohémagglutinine à partir du jour 4 ou 5.

Par conséquent, l'expression du gène LAG-2 est induite par la stimulation antigénique ou mitogénique des lymphocytes T ou NK. L'apparition de l'expression de ce gène est assez tardive après activation (jour 4 à 5). L'expression est conservée sur de longues périodes (plusieurs mois) lorsque les lignées lymphocytaires sont mises en culture avec l'interleukine-2.

### VIII - Analyses du type Southern blot du gène LAG-2

L'ADN génomique des lignées K562, Laz 388 et Jurkat a été digéré avec des enzymes de restriction. L'ADN digéré a été migré sur gel d'agarose 0,7 % et transféré sur membrane de nylon tel que décrit par Triebel et al. (8). Les filtres ont ensuite été hybridés avec la sonde FC24 comme décrit par Triebel et al. (8). Les mêmes fragments de restriction hybridant avec la sonde FC24 ont été retrouvés après digestion avec EcoRI, BamHI ou HindIII, ce qui montre que tout l'ADN a été extrait. Avec EcoRI, on note 3 bandes caractéristiques à environ 2 kb, 3,3 kb et 5,2 kb. Ce sont ces 3 bandes qui, une fois clonées, serviront à établir la structure moléculaire du gène LAG-2 (voir paragraphe suivant). L'analyse de différents fragments de restriction a montré que le gène LAG-2 était très probablement unique et présent à une seule copie par génome haploïde.

### IX - Isolement du gène LAG-2 et étude de sa région promotrice

On isole des clones d'ADN génomique à partir de la banque LY67 obtenue à partir d'ADN d'une lignée de cellules B humaines transformées par l'EBV, digérée partiellement par Mbo1 et insérée dans le phage lambda 2001 comme décrit par Dariavach et al. (10). On marque le fragment de restriction FC24 par la méthode des amorces avec hexamères aléatoires décrite par Feinberg (11) et on l'utilise par cribler 2 x 10⁵ plaques de la banque d'ADN de génome humain. On isole ainsi 6 clones appelés GC1 à GC6 et on caractérise les ADN des phages par des cartes de restriction à l'aide de la sonde FC24. Tous les clones donnent la même carte de restriction après hybridation. A partir du clone GC1, 3 fragments EcoRI hybridant avec la sonde FC24 sont isolés; ces fragments font 2 kb, 3,4 kb et 5,2 kb. Ils sont clonés dans le site EcoRI du plasmide pBS.

On construit ensuite des cartes de restriction détaillées de ces sous clones et on compare les résultats obtenus avec la carte de restriction de la séquence FC24 définie plus haut. De nombreux fragments sont obtenus sur gel d'agarose à point de gélification bas; ils sont sous clonés ensuite dans les bactériophages M13mp18, M13mp19. Les séquences de ces fragments sont obtenues à partir d'ADN simple brin à l'aide de la technique de terminaison de chaîne dideoxy décrite par Sanger et al. (9).

Ainsi ont pu être définies les limites exon-intron portant sur les 3 premiers exons ainsi que la séquence de la région promotrice. L'exon 1 comprend les nucléotides 2 à 76; l'exon 2, les nucléotides 77 à 179 et l'exon 3, les nucléotides 180 à 278 de la séquence FC24 décrite plus haut. Il est à noter que le nucléotide en position +1 de la séquence FC24 n'est pas retrouvé à l'échelon génomique et il est possible que ce nucléotide (un C dans la séquence FC24) appartienne au linker EcoRI qui a servi au clonage dans la banque d'ADNc. Il est retrouvé un A à la position correspondante dans la séquence génomique (position 680 de la séquence montrée aux pages 5 et 6). La séquence des exons est identique à la séquence du clone FC24 décrit plus haut et indique donc qu'il y a peu de polymorphisme génétique entre différents individus puisque la banque d'ADNc dont est issu le clone FC24 d'une part, et la banque génomique LY67, d'autre part, sont issues de matériel cellulaire provenant de deux individus différents.

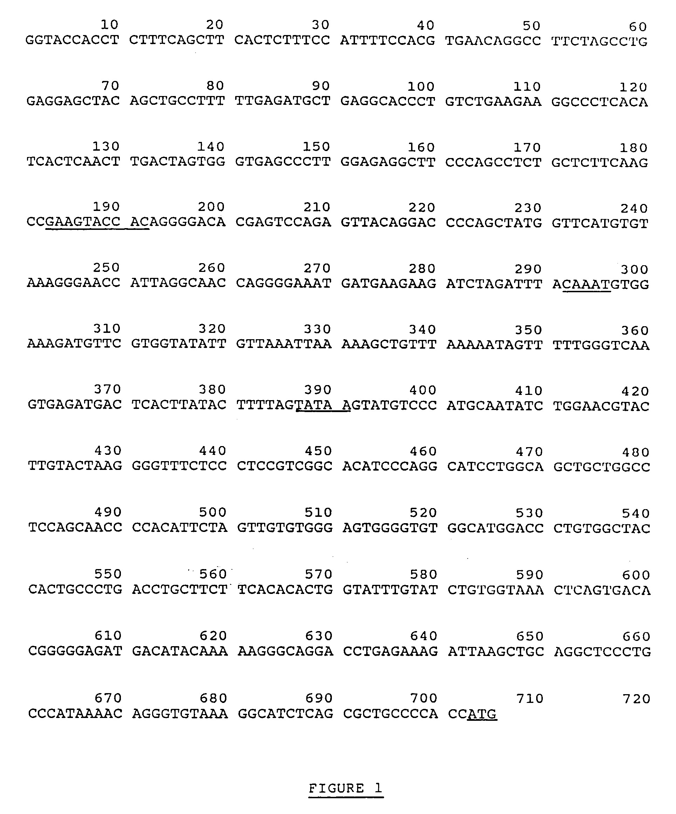

La séquence promotrice montrée à la Fig. 1 comprend 705 nucléotides, les 3 derniers correspondant aux nucléotides A, T et G (codon d'initiation de la traduction). Cette séquence correspond aux 702 nucléotides présents au niveau du gène LAG-2 en position 5′ du site d'initiation de la traduction (ATG) défini par l'analyse de l'ADNc FC24. Les sites de fixation des promoteurs possibles (soulignés) sont : 1/ une boîte TATA en position 386; 2/ une boîte CCAAT en position 291; 3/ un décanucléotide en type GM-CSF (séquence consensus GAAATTCAC) en position 183 (séquence GAAGTACCAC). Les deux premiers sites (TATA et CCAAT) sont retrouvés de façon ubiquitaire dans les promoteurs des gènes eucaryotes. Le dernier site (décanucléotide de type GM-CSF) a été décrit comme étant une séquence relativement spécifique des cytokines, retrouvée dans la partie 5′ du gène codant pour le GM-CSF et du gène codant pour l'IL-4 chez l'homme (12). 4/ Enfin, un site de fixation pour la protéine AP-1 séquence T G A C T C A est présent en position 367 à 373 (14 et 15).

### X - Transcription in vitro et traduction du gène LAG-2

Afin de déterminer de façon définitive la région codante et la capacité d'être traduit de l'ARNm dérivé par transcription du clone FC24, nous avons transcrit les 2 brins de l'ADNc FC24 utilisant soit la T3, soit la T7 polymérase et comme substrat le clone FC24 dans le vecteur pBS. Les deux préparations d'ARN ont ensuite été traduites in vitro en utilisant un extrait de réticulocytes de lapin en présence de méthionine ³⁵S. L'ARN sens contenant la queue poly-A du côté 3′ a été traduit en une protéine de masse moléculaire d'environ 16.000 daltons détectable en autoradiographie après migration sur gel de type SDS-PAGE. Cette masse moléculaire estimée est très semblable à la masse moléculaire théorique de 16.380 correspondant aux 145 acides aminés du polypeptide LAG-2 avec un signal peptide intact, non clivé. Aucun produit de translation n'a pu être détecté en utilisant l'ARN anti-sens comme substrat dans la réaction avec l'extrait de réticulocyte de lapin.

### XI - Expression de la protéine LAG-2 recombinante

Nous avons employé un système utilisant un vecteur de type "baculovirus". Ce système permet de produire des protéines étrangères dans des cellules d'insecte, par exemple des cellules SF9 (dépôt ATCC CRRL 1711), en utilisant une recombinaison in vivo entre un vecteur de transfert qui contient le gène étranger d'une part et d'autre part, le génome d'un virus. Après transfection du plasmide recombinant et du génome du virus, on sélectionne par purifications successives (criblage des recombinants) les cellules SF9 permettant d'obtenir une production de protéine recombinante importante. Cette protéine est normalement clivée (le peptide signal hydrophobe est enlevé à l'intérieur de la cellule) et glycosylée (au moins partiellement).

### Détection de la protéine recombinante :

Un peptide synthétique de 24 acides aminés correspondant à la séquence NH₂ terminale déduite de la traduction du clone d'ADN FC24 a été synthétisé. Ce peptide a ensuite été couplé à une protéine porteuse, la KLH ("Keyhole Lympet Hemocyanin"). Des immunisations répétées à des lapins ont permis d'obtenir un sérum réagissant au 1/10000ème dans des tests de type ELISA avec ce peptide synthétique. La fraction immunoglobuline de ces sérums de lapin a été purifiée sur colonne de protéine A - Sépharose.

Des surnageants de cellules SF9 infectées par le clone recombinant de virus contenant le fragment FC24 ont été obtenus au 3ème jour de la culture et ont été testés par la technique dite du "Western blot" avec les anticorps purifiés anti-peptidiques anti-FC24 décrits plus haut. On a ainsi obtenu un signal net correspondant à une protéine d'environ 14 kd après révélation avec des anticorps de chèvre anti-lapin marqués à la peroxydase.

### XII - Analyse de la protéine LAG-2 recombinante

### 1) Purification sur colonne d'affinité et analyse N-terminal de la protéine recombinante

Les anticorps de lapin anti-peptide NH₂-terminal de l'ADNc LAG-2 ont été purifiés par passage sur Protéine A - Sépharose. Ces anticorps purifiés ont ensuite été couplés sur CH-sépharose active à raison de 5 µg d'anticorps par ml de gel. Les tampons dilution utilisés pour la colonne d'immuno-affinité sont les suivants :
- Tampon 20mM TRIS 10 % éthylène glycol 0,1 % N-octyl glucopyrannoside pH 7,5 (tampon de charge)
- Tampon 20mM TRIS 10 % éthylène glycol 0,1 % N-octyl glucopyrannoside 0,25 M NaCl pH 7,5 (tampon de lavage)
- Tampon 50mM glycine pH 2,5 (tampon d'élution)
- Tampon 50mM triéthylamine pH 11 (tampon de régénération)
- Tampon 20mM TRIS base 2,5 % mannitol (pH spontané envir. 9)
- Tampon 20mM TRIS 10 % ethylène glycol 0,1 % N-octyl glucopyrannoside 0,01 % azoture de sodium pH 7,5 (tampon de conservation de l'immuno-absorbant)

### 2) Mode opératoire

- 160 ml de surnageant de culture de cellules SF9 infectées par baculovirus ayant intégré l'ADNc FC24 (clone 5-01-A6) sont dilués au 1/2 en tampon de charge et chargés sur 5 ml d'immuno-absorbant avec un débit de 0,7 ml/mm (vitesse linéaire = 9,5 cm/h) à température ambiante
- le gel est ensuite lavé avec le tampon de charge jusqu'à ce que la densité optique à 280 se stabilise (environ 0,080 AU)
- le gel est lavé avec le tampon de lavage (0,25 M NaCl) jusqu'à D.O. environ 0
- les protéines fixées sont éluées au moyen du tampon d'élution froid : l'éluat est recueilli dans un égal volume de tampon 20 mN TRIS 25 mg/ml mannitol
- la colonne est lavée par le tampon de régénération (environ 10 volumes de gel) puis est rééquilibrée en tampon de charge. Elle est enfin conditionnée à 4 C en tampon de conservation.

### 3) Analyse des éluats en SDS-PAGE

200 ul de chaque tube d'éluat sont concentrés à sec après avoir été neutralisés par 10 µl de TRIS-base 1 M. Les échantillons sont repris dans 20 µl de tampon d'échantillon d'électrophorèse et 1 ul de cet échantillon est déposé sur gel. Le gel SDS est ensuite coloré au bleu de Coomasie. On estime la quantité de la protéine LAG-2 (bande à 14 kd) à environ 500 ng à 1 µg/ml d'éluat.

### 4) Séquençage N-terminal après SDS-PAGE et transfert sur membrane

### a) SDS PAGE

L'électrophorèse des protéines est faite selon la méthode décrite par Shagger et Von Jagow (Anal. Biochem., 166:368-379, 1987).

### b) Transfert et microséquence

La technique utilisée est celle decrite par P. MATSUDAIRA (JBC, vol. 262, 21:10035-10038, 1987). Après SDS PAGE ou transfert sur membrane PVDF et découpage du spot de 14 kd, on analyse la séquence des 20 premiers amino-acides par la méthode automatique de dégradation d'EDMAN au moyen d'un séquenceur ABI 470A. Les résultats sont:

| CYCLE | AMINO-ACIDE |
|---|---|
| 1 | Arg Tyr Ser Pro Met Val Ala Gly Glu |
| 2 | Leu |
| 3 | Ser |
| 4 | ? |
| 5 | Glu |
| 6 | Tyr |
| 7 | Tyr |
| 8 | Asp Gly |
| 9 | Leu |
| 10 | Ala |
| 11 | ? |
| 12 | Ala |
| 13 | ? |
| 14 | Leu |
| 15 | ? |
| 16 | Asp |
| 17 | Glu |
| 18 | Glu |
| 19 | Lys |
| 20 | Ser |

Il est à noter que l'indétermination du premier amino-acide est habituelle dans la technique utilisée.

### Conclusion :

Cette séquence correspond bien à la séquence de la protéine LAG-2 déduite de la traduction du clone FC24 décrit plus haut et indique que le signal peptide est clivé entre la position 21 et la position 22. L'arginine est donc l'amino-acide NH₂ terminal de la protéine LAG-2 mature.

### XIII - Immunoprécipitation de la protéine LAG-2 naturelle

Les immunoprécipitations ont été faites après marquage interne des cellules F55IIIE5 (d'où est originaire le clone d'ADNc FC24) et comme contrôle la cellule B, EBV-transformée, Laz 388. Les protéines des cellules (3 x 10⁷ cellules) ont été marquées avec la méthionine ³⁵S (200 µCi/ml) pendant 4 heures à la concentration de 10⁷ cellules/ml. Après marquage, le surnageant des cellules a été obtenu par centrifugation et dilué 5 fois avec un tampon phosphate (pH 7,2) 0,01 M contenant 1 % de triton X-100 et 0,1 % de Na-desoxycholate. Les immunoprécipitations ont été réalisées avec les hétéro-anticorps de lapin couplés à des billes de Sépharose - Protéine A tel que décrit par Moingeon et al. (13). Les protéines LAG-2 immunoprécipitées ont été dissociées des billes de Sépharose 4B par une incubation en tampon de charge SDS (3 % de SDS à 100° pendant 3 minutes) et soumis à un gel de polyacrylamide SDS (15 % d'acrylamide) en conditions réduites et non réduites (5 % de β-mercaptoéthanol). En conditions réduites et non réduites, on obtient une bande à environ 15 kd après autoradiographie. Cette bande est retrouvée dans le surnageant de la cellule F55IIIE5 mais pas dans le surnageant contrôle de la cellule Laz 388. Ces expériences indiquent donc que la protéine LAG-2 est bien sécrétée de façon naturelle par les cellules activées humaines. D'autre part, le poids moléculaire retrouvé est semblable au poids moléculaire de la protéine recombinante produite dans le système dit du baculo-virus.

### REFERENCES

1. Nowill, A., P. Moingeon, A. Ythier, M. Graziani, F. Faure, L. Delmon, M. Rainaut, F. Daffos, C. Bohuon and T. Hercend, 1986. Natural killer clones derived from fetal (25 wk) blood : probing the human T cell receptor with WT31 monoclonal antibody. J. Exp. Med. 163: 1601.
2. Maniatis, T., E.F. Fritsch and J. Szmbrook 1982. Molecular cloning : A laboratory manual, Cold spring harbor laboratory New York.
3. Mechler, B., and T.H. Rabbitts 1981. Membrane-bound ribosomes of myeloma celles IV. mRNA complexity of free and membrane-bound polysomes. J. Cell Biol. 88:29.
4. Aviv, H., and P. Leder, 1972. Purification of biologically active globin messenger RNA by chromatography on oligothymidilic acid cellulose. Proc. Natl. Acad. Sci. USA 69 : 1408.
5. Gubler, U., and B.J. Hoffman, 1983. A simple and very efficient method for generating cDNA librairies. Gene. 25 : 263.
6. Huynh, T.V., R.A. Young and R.W. Davis, 1985. DNA cloning : A practical approach. 49-78, D. Glover Editor. IRL Press. Oxford. United Kingdom.
7. Davis, M.M., D.I. Cohen, E.A. Nielsen, M. Steinmetz, W.E. Paul and I. Hood, 1984. Cell-type specific cDNA probes and the murine I region : the localization and orientation of A a. Proc. Natl. Acad. Sci. USA. 81:2194.
8. Triebel, F., M. Graziani, F. Faure, S. Jitsukawa and T. Hercend. 1987. Cloned human CD3 - lymphocytes with NK-like activity do not express nor rearrange TCR gamma genes. Eur. J. Immunol. 17:1209.
9. Sanger, F., S. Nicklen and A.R. Coulson, 1977. DNA sequencing with chain-terminating inhibitors. Proc. Natl. Acad. Sci. USA 75:5463.
10. Dariavach, P., G. Lefranc and M.P. Lefranc, 1987. Human Immunoglobulin Clambda6 gene encodes the Kern/Oz-lambda chain and Clambda4 and Clambda5 are pseudogenes, Proc. Natl. Acad. Sci. USA 84:9074.
11. Feinberg, A.P., and B. Vogelstein. 1983. A technique for radiolabeling DNA restriction endonuclease fragments to hugh specific activity, Anal. Biochem. 132:6.
12. Arai, N., Nomura, D., Villaret, D., De Waal Malefijt, R., Seiki, M., Yoshida, M., Minoshima, S., Fukuyama, R., Maekawa, M., Kudoh, J., Shimizu, N., Yokata, K., Abe, E., Yokota T., & Takebe, Y. (1989). Complete nucleotide sequence of the chromosomal gene for human IL-4 and its expression. The Journal of Immunology, 142, 274-282.
13. Moingeon, P., S. Jitsukawa, F. Faure, F. Troalen, F. Triebel, M. Graziani, F. Forestier, D. Bellet, C. Bohuon and T. Hercend. 1987. A gamma-chain complex forms a functional receptor on cloned human lymphocytes with natural killer-like activity. Nature. 325:723.
14. Angel, P., Imagawa, M., Chiu, R., Stein, B. Imbra, R. Rhamsdorf, H.J. Jonat, C. Herrlich, P. & Karin, M. (1987) Cell 49, 729-739.
15. Lee, W., Mitchell, P. & Tjian, R. (1987) Cell 49, 741-752.

| Symboles des acides aminés | | |
|---|---|---|
| A | Ala | alanine |
| C | Cys | cytéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | Thr | thréonine |
| V | Val | valine |
| W | Trp | tryptophane |
| Y | Tyr | tyrosine |

## Revendications (Revendications pour l'(les) Etat(s) contractant(s) suivant(s): AT, BE, CH, DE, FR, GB, GR, IT, LU, NL, SE)

1. Lymphokines constituées par la séquence peptidique : 
X - R L S  P E Y Y D  L A R A H  L R D E E  K S C P C
L A Q E G  P Q G D L  L T K T Q  E L G R D  Y R T C L
T I V Q K  L K K M V  D K P T Q  R S V S N  A A T R V
C T R G R  S R W R D  V C R N F  M R R Y Q  S R V I Q
G L V A G  E T A Q Q  I C E D L  R L C I P  S T G P L
 dans laquelle X est choisi parmi H, un reste méthionine ou la séquence :

M A T W A  L L L L A  A M L L G  N P G L V  F S

2. Séquence d'ADN comprenant une séquence d'ADN codant pour une lymphokine selon la revendication 1.

3. Séquence d'ADN codant pour une lymphokine selon la revendication 1.

4. Séquence d'ADN selon la revendication 3 comprenant essentiellement la séquence

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

5. Composition pharmaceutique comprenant à titre de principe actif une lymphokine selon la revendication 1.

6. Anticorps monoclonaux dirigés spécifiquement contre une lymphokine selon la revendication 1.

7. Hybridomes produisant des anticorps monoclonaux selon la revendication 6.

8. Plasmide contenant une séquence d'ADN codant pour une lymphokine selon la revendication 1.

9. Vecteur d'expression comprenant une séquence d'ADN codant pour une lymphokine selon la revendication 1.

10. Hôte transformé avec un vecteur selon la revendication 9.

11. Hôte transformé par un vecteur d'expression comprenant comme séquence d'ADN promotrice tout ou partie de la séquence 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC

490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA

610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 et une séquence d'ADN codant pour une lymphokine selon la revendication 1 pour l'expression de ladite lymphokine sous le contrôle de ladite séquence promotrice.

## Revendications (Revendications pour l'(les) Etat(s) contractant(s) suivant(s): ES)

1. Procédé de préparation de lymphokines constituées par la séquence peptidique : 
X - R L S  P E Y Y D  L A R A H  L R D E E  K S C P C
L A Q E G  P Q G D L  L T K T Q  E L G R D  Y R T C L
T I V Q K  L K K M V  D K P T Q  R S V S N  A A T R V
C T R G R  S R W R D  V C R N F  M R R Y Q  S R V I Q
G L V A G  E T A Q Q  I C E D L  R L C I P  S T G P L
 dans laquelle X est choisi parmi H, un reste méthionine ou la séquence : M A T W A  L L L L A  A M L L G  N P G L V  F S caractérisé en ce qu'elles sont obtenues par synthèse peptidique classique ou par insetion d'une séquence d'ADN codant pour lesdites lymphokines dans un vecteur d'expression et la tranfection de cellules avec ce vecteur d'expression.

2. Procédé selon la revendication 1, caractérisé en ce que la séquence d'ADN comprend essentiellement la séquence

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

3. Procédé de préparation d'une composition pharmaceutique, caractérisé en ce que l'on utilise à titre de principe actif une lymphokine selon la revendication 1.

4. Procédé de préparation d'anticorps monoclonaux, caractérisé en ce qu'on les prépare au moyen d'une lymphokine préparée selon le procédé de la revendication 1.

5. Procédé de préparation d'un hybridome produisant des anticorps monoclonaux, caractérisé en ce qu'on le prépare au moyen d'une lymphokine préparée selon le procédé de la revendication 1.

6. Procédé de préparation d'un plasmide, caractérisé en ce qu'il comprend une séquence d'ADN codant pour une lymphokine selon la revendication 1.

7. Procédé de préparation d'un vecteur d'expression, caractérisé en ce qu'il comprend une séquence d'ADN codant pour une lymphokine préparée selon le procédé de la revendication 1.

8. Procédé de préparation d'un hôte transformé, caractérisé en ce qu'on utilise un vecteur préparé selon la revendication 7.

9. Procédé de préparation d'un hôte transformé par un vecteur d'expression, caractérisé en ce qu'on utilise comme séquence d'ADN promotrice tout ou partie de la séquence 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC

490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA
 
610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 et une séquence d'ADN codant pour une lymphokine préparée selon la revendication 1 pour l'expression de ladite lymphokine sous le contrôle de ladite séquence promotrice.

## Claims (Claims for the following Contracting State(s): AT, BE, CH, DE, FR, GB, GR, IT, LU, NL, SE)

1. Lymphokines constituted by the peptide sequence : 
X - R L S   P E Y Y D   L A R A H   L R D E E   K S C P C
L A Q E G   P Q G D L   L T K T Q   E L G R D   Y R T C L
T I V Q K   L K K M V   D K P T Q   R S V S N   A A T R V
C T R G R   S R W R D   V C R N F   M R R Y Q   S R V I Q
G L V A G   E T A Q Q   I C E D L   R L C I P   S T G P L
 in which X is selected from H, a methionine residue or the sequence :

MATWA LLLLA AMLLG NPGLV FS

2. DNA sequence comprising a DNA sequence coding for a lymphokine according to claim 1.

3. DNA sequence coding for a lymphokine according to claim 1.

4. DNA sequence according to claim 3 comprising essentially the sequence

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

5. Pharmaceutical composition comprising as an active ingredient a lymphokine according to claim 1.

6. Monoclonal antibodies directed specifically against a lymphokine according to claim 1.

7. Hybridomas producing monoclonal antibodies according to claim 6.

8. Plasmid containing a DNA sequence coding for a lymphokine according to claim 1.

9. Expression vector comprising a DNA sequence coding for a lymphokine according to claim 1.

10. Transformed host with a vector according to claim 9.

11. Host transformed by an expression vector comprising as the promoter DNA sequence all or part of the sequence 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC

490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA

610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 and a DNA sequence coding for a lymphokine according to claim 1 for the expression of the said lymphokine under the control of the said promoter sequence.

## Claims (Claims for the following Contracting State(s): ES)

1. Process for the preparation of lymphokines consisting of the peptide sequence : 
X - R L S   P E Y Y D   L A R A H   L R D E E   K S C P C
L A Q E G   P Q G D L   L T K T Q   E L G R D   Y R T C L
T I V Q K   L K K M V   D K P T Q   R S V S N   A A T R V
C T R G R   S R W R D   V C R N F   M R R Y Q   S R V I Q
G L V A G   E T A Q Q   I C E D L   R L C I P   S T G P L
 in which X is selected from H, a methionine residue or the sequence : M A T W A   L L L L A   A M L L G   N P G L V   F S characterized in that they are obtained by standard peptide synthesis or by insertion of a DNA sequence coding for the said lymphokines in an expression vector and transfection of cells with this expression vector.

2. Process according to claim 1, characterized in that the DNA sequence comprises essentially the sequence :

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

3. Process for preparing a pharmaceutical composition, characterized in that a lymphokine according to claim 1 is used as the active ingredient.

4. Process for preparing monoclonal antibodies, characterized in that they are prepared by means of a lymphokine prepared according to the process of claim 1.

5. Process for preparing a hybridoma producing monoclonal antibodies, characterised in that it is prepared by means of a lymphokine prepared according to the process of claim 1.

6. Process for preparing a plasmid, characterized in that it comprises a DNA sequence coding for a lymphokine according to claim 1.

7. Process for preparing an expression vector, characterized in that it comprises a DNA sequence coding for a lymphokine prepared according to the process of claim 1.

8. Process for preparing a transformed host, characterized in that use is made of a vector prepared according to claim 7.

9. Process for preparing a transformed host by an expression vector, characterized in that, as a promoter DNA sequence, use iS made of all or part of the sequence 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC
490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA
 
610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 and a DNA coding sequence for a lymphokine prepared according to claim 1 for the expression of the said lymphokine under the control of the said promoter sequence.

## Patentansprüche (Patentansprüche für folgende(n) Vertragsstaat(en): AT, BE, CH, DE, FR, GB, GR, IT, LU, NL, SE)

1. Lymphokine, bestehend aus der Peptidsequenz 
X - R L S  P E Y Y D  L A R A H  L R D E E  K S C P C
L A Q E G  P Q G D L  L T K T Q  E L G R D  Y R T C L
T I V Q K  L K K M V  D K P T Q  R S V S N  A A T R V
C T R G R  S R W R D  V C R N F  M R R Y Q  S R V I Q
G L V A G  E T A Q Q  I C E D L  R L C I P  S T G P L
 in der X ausgewählt ist aus H, einem Methioninrest oder der Sequenz

M A T W A  L L L L A  A M L L G  N P G L V  F S

2. DNA-Sequenz, die eine für ein Lymphokin nach Anspruch 1 kodierende DNA-Sequenz umfaßt.

3. Für ein Lymphokin nach Anspruch 1 kodierende DNA-Sequenz.

4. DNA-Sequenz nach Anspruch 3, die im wesentlichen die folgende Sequenz umfaßt:

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

5. Pharmaseutische Zusammensetzung, die als Wirkstoff ein Lymphokin nach Anspruch 1 enthält.

6. Monoklonale Antikörper, die spezifisch gegen ein Lymphokin nach Anspruch 1 gerichtet sind.

7. Hybridome, die monoklonale Antikörper nach Anspruch 6 erzeugen.

8. Plasmid, das eine für ein Lymphokin nach Anspruch 1 kodierende DNA-Sequenz enthält.

9. Expressionsvektor, der eine für ein Lymphokin nach Anspruch 1 kodierende DNA-Sequenz umfaßt.

10. Wirt, der durch einen Vektor nach Anspruch 9 transformiert ist.

11. Wirt, der durch einen Expressionsvektor transformiert ist, der als DNA-Promotor-Sequenz die Sequenz 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA

370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC

490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA

610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 ganz oder teilweise und eine für ein Lymphokin nach Anspruch 1 kodierende DNA-Sequenz für die Expression dieses Lymphokins unter der Steuerung dieser Promotor-Sequenz aufweist.

## Patentansprüche (Patentansprüche für folgende(n) Vertragsstaat(en): ES)

1. Verfahren zur Herstellung von Lymphokinen, die aus der Peptidsequenz 
X - R L S   P E Y Y D   L A R A H   L R D E E   K S C P C
L A Q E G   P Q G D L   L T K T Q   E L G R D   Y R T C L
T I V Q K   L K K M V   D K P T Q   R S V S N   A A T R V
C T R G R   S R W R D   V C R N F   M R R Y Q   S R V I Q
G L V A G   E T A Q Q   I C E D L   R L C I P   S T G P L
 bestehen, in der X ausgewählt ist aus H, einem Methioninrest oder der Sequenz: MATWA LLLLA AMLLG NPGLV FS dadurch gekennzeichnet, daß sie durch bekannte Peptidsynthese oder durch Insertion einer für diese Lymphokine kodierenden DNA-Sequenz in einen Expressionsvektor und Transformation von Zellen mit diesem Expressionsvektor erhalten werden.

2. Verfahren nach Anspruch 1, dadurch gekennzeichnet, daß die DNA-Sequenz im wesentlichen die folgende Sequenz umfaßt:

10 20 30 40 50 60
CGGCATCTCA GCGGCTGCCC CACCATGGCT ACCTGGGCCC TCCTGCTCCT TGCAGCCATG

70 80 90 100 110 120
CTCCTGGGCA ACCCAGGTCT GGTCTTCTCT CGTCTGAGCC CTGAGTACTA CGACCTGGCA

130 140 150 160 170 180
AGAGCCCACC TGCGTGATGA GGAGAAATCC TGCCCGTGCC TGGCCCAGGA GGGCCCCCAG

190 200 210 220 230 240
GGTGACCTGT TGACCAAAAC ACAGGAGCTG GGCCGTGACT ACAGGACCTG TCTGACGATA

250 260 270 280 290 300
GTCCAAAAAC TGAAGAAGAT GGTGGATAAG CCCACCCAGA GAAGTGTTTC CAATGCTGCG

310 320 330 340 350 360
ACCCGGGTGT GTAGGACGGG GAGGTCACGA TGGCGCGACG TCTGCAGAAA TTTCATGAGG

370 380 390 400 410 420
AGGTATCAGT CTAGAGTTAT CCAGGGCCTC GTGGCCGGAG AAACTGCCCA GCAGATCTGT

430 440 450 460 470 480
GAGGACCTCA GGTTGTGTAT ACCTTCTACA GGTCCCCTCT GAGCCCTCTC ACCTTGTCCT

490 500 510 520 530 540
GTGGAAGAAG CACAGGCTCC TGTCCTCAGA TCCCGGGAAC GTCAGCAACC TCTGCCGGCT

550 560 570 580 590 600
CCTCGCTTCC TCGATCCAGA ATCCACTCTC CAGTCTCCCT CCCCTGACTC CCTCTGCTGT

610 620 630 640 650 660
CCTCCCCTCT CAGGAGAATA AAGTGTCAAG CAAG

3. Verfahren zur Herstellung einer pharmazeutischen Zusammensetzung, dadurch gekennzeichnet, daß man als Wirkstoff ein Lymphokin nach Anspruch 1 verwendet.

4. Verfahren zur Herstellung von monoklonalen Antikörpern, dadurch gekennzeichnet, daß man sie mit Hilfe eines in dem Verfahren von Anspruch 1 hergestellten Lymphokins herstellt.

5. Verfahren zur Herstellung eines monoklonale Antikörper erzeugenden Hybridoms, dadurch gekennzeichnet, daß man es mit Hilfe eines in dem Verfahren von Anspruch 1 hergestellten Lymphokins herstellt.

6. Verfahren zur Herstellung eines Plasmids, dadurch gekennzeichnet, daß es eine für ein Lymphokin nach Anspruch 1 kodierende DNA-Sequenz besitzt.

7. Verfahren zur Herstellung eines Expressionsvektors, dadurch gekennzeichnet, daß er eine DNA-Sequenz besitzt, die für ein in dem Verfahren von Anspruch 1 hergestelltes Lymphokin kodierend ist.

8. Verfahren zur Herstellung eines transformierten Wirts, dadurch gekennzeichnet, daß man einen gemäß Anspruch 7 hergestellten Vektor verwendet.

9. Verfahren zur Herstellung eines durch einen Expressionsvektor transformierten Wirts, dadurch gekennzeichnet, daß man als DNA-Promotor-Sequenz die Sequenz 
10 20 30 40 50 60
GGTACCACCT CTTTCAGCTT CACTCTTTCC ATTTTCCACG TGAACAGGCC TTCTAGCCTG

70 80 90 100 110 120
GAGGAGCTAC AGCTGCCTTT TTGAGATGCT GAGGCACCCT GTCTGAAGAA GGCCCTCACA

130 140 150 160 170 180
TCACTCAACT TGACTAGTGG GTGAGCCCTT GGAGAGGCTT CCCAGCCTCT GCTCTTCAAG

190 200 210 220 230 240
CCGAAGTACC ACAGGGGACA CGAGTCCAGA GTTACAGGAC CCCAGCTATG GTTCATGTGT

250 260 270 280 290 300
AAAGGGAACC ATTAGGCAAC CAGGGGAAAT GATGAAGAAG ATCTAGATTT ACAAATGTGG

310 320 330 340 350 360
AAAGATGTTC GTGGTATATT GTTAAATTAA AAAGCTGTTT AAAAATAGTT TTTGGGTCAA
 
370 380 390 400 410 420
GTGAGATGAC TCACTTATAC TTTTAGTATA AGTATGTCCC ATGCAATATC TGGAACGTAC

430 440 450 460 470 480
TTGTACTAAG GGGTTTCTCC CTCCGTCGGC ACATCCCAGG CATCCTGGCA GCTGCTGGCC

490 500 510 520 530 540
TCCAGCAACC CCACATTCTA GTTGTGTGGG AGTGGGGTGT GGCATGGACC CTGTGGCTAC

550 560 570 580 590 600
CACTGCCCTG ACCTGCTTCT TCACACACTG GTATTTGTAT CTGTGGTAAA CTCAGTGACA

610 620 630 640 650 660
CGGGGGAGAT GACATACAAA AAGGGCAGGA CCTGAGAAAG ATTAAGCTGC AGGCTCCCTG

670 680 690 700 710 720
CCCATAAAAC AGGGTGTAAA GGCATCTCAG CGCTGCCCCA CCATG
 ganz oder teilweise und eine DNA-Sequenz, die für ein gemäß Anspruch 1 hergestelltes Lymphokin kodierend ist, für die Expression dieses Lymphokins unter der Steuerung dieser Promotor-Sequenz verwendet.
